# EUROPÄISCHE PATENTSCHRIFT

(11) **EP 4 183 357 B1**
(45) Veröffentlichungstag und Bekanntmachung des Hinweises auf die Patenterteilung: **19.03.2025**
(21) Anmeldenummer: 21209026.0
(22) Anmeldetag: 18.11.2021
(51) Int. Cl.: A61B 18/14

(54) **MEDIZINISCHES INSTRUMENT**
MEDICAL INSTRUMENT
INSTRUMENT MÉDICAL

(43) Veröffentlichungstag der Anmeldung: 24.05.2023
(73) Patentinhaber: Erbe Elektromedizin GmbH, 72072 Tübingen (DE)
(72) Erfinder: MÜLLER, Marc, 72074 Tübingen (DE); KLEBER, Frederik, 72827 Wannweil (DE)
(74) Vertreter: Rüger Abel Patentanwälte PartGmbB

(56) Entgegenhaltungen:
- WO-A1-96/01132
- AU-B2- 2019 204 666
- US-A1- 2012 289 954
- US-A1- 2020 237 421

## Beschreibung

Die Erfindung betrifft ein medizinisches Instrument zur medizinischen oder chirurgischen Behandlung eines menschlichen oder tierischen Patienten. Insbesondere betrifft die Erfindung ein solches Instrument, das eine Lichtleiteinrichtung enthält, die dazu eingerichtet ist, Licht von einem Wirkort des Instruments fortzuleiten oder, zusätzlich oder alternativ, Licht zu einem Wirkort des Instruments hinzuleiten.

Aus der EP 2 113 190 ist eine Anordnung, bestehend aus einem chirurgischen Instrument und einem Trokar bekannt, durch dessen Arbeitskanal das Instrument geschoben werden kann. Das Instrument enthält eine Lichtleiteinrichtung, mittels derer Licht am distalen Ende des Instruments aufgenommen oder abgegeben werden kann. Der Trokar ist an seinem distalen Ende mit einem oder mehreren Schutzelementen versehen, die den Arbeitskanal versperren und so das Eindringen von Fluiden in den Arbeitskanal verhindern. Das Instrument kann, wenn es in distaler Richtung bewegt wird, das Schutzelement beiseite schieben, um auf diese Weise freie Sicht auf das Zielgebiet des Instruments erhalten.

Die EP 3 195 824 A1 schlägt ein Instrument mit einem Kanal vor, in welchem eine optische Faser längsbeweglich angeordnet ist. Die optische Faser weist an ihrem distalen Ende eine lichtemittierende Fläche auf, aus der ein Laserstahl austreten kann. Der Kanal ist an seinem distalen Ende mit einem Wischelement 21 versehen, das den Kanal bei rückgezogener Faser verschließt und das Eindringen von feinen Fluidtropfen in den Kanal und den Niederschlag derselben aus der Lichtaustrittsfläche der Faser verhindert. Beim Vorschieben der Faser wischt das Wischelement die Lichtaustrittsfläche ab und säubert diese somit.

Weiterer Stand der Technik wird durch die EP 1 773 223 B1 gebildet, die eine Einrichtung zur Argon-Plasma -Koagulation veranschaulicht, deren gasführender Kanal mit Verschlusselementen versehen ist. Aus der WO 96/01132 A1 ist ein Instrument mit länglichem Schaft bekannt, an dessen Ende ein Verschlusselement sitzt. Durch den hohl ausgebildeten Schaft kann ein optisches längliches Element geschoben werden, dass zur Betrachtung eines vor der Spitze des Instruments liegenden Bereichs eingerichtet ist. Das optische Element kann axial bewegt werden und dazu aus einer distalen Öffnung des Instruments herausgeschoben oder in diese zurückgezogen werden. Zum Verschluss der Öffnung dienen flexible Flügel, die in entspanntem Zustand aneinander anliegen und von dem optischen stabförmigen Element auseinander gedrängt werden können.

Es existieren Anwendungsfälle, bei denen die Lichtleiteinrichtung oder ein sonstiges optisches Element eines Instruments unbeweglich gelagert sein soll oder muss, sodass es nicht aus einem Verschmutzungsbereich herausbewegt werden kann.

Davon leitet sich die der Erfindung zugrundeliegende Aufgabe ab, wonach eine Möglichkeit geschaffen werden soll, ein optisches Element eines Instruments bei Benutzung desselben möglichst sauber zu halten.

Diese Aufgabe wird mit dem Instrument nach Anspruch 1 gelöst:
Das erfindungsgemäße Instrument weist einen Instrumentenkörper auf, in dem mindestens ein Kanal ausgebildet ist, der ein distales Ende und ein proximales Ende aufweist. Alternativ kann ein solcher Kanal auch "an" dem Instrument ausgebildet sein, indem er in einem gesonderten Element ausgebildet ist, das mit dem Körper des Instruments z.B. durch eine oder mehrere Klemmen (z.B. federnde Clips) verbunden ist. Die nachfolgende Beschreibung gilt für beide Ausführungen.

Der Kanal kann sich insbesondere von dem distalen Ende des Instruments bis zu dessen proximalen Ende erstrecken, an dem eine Anschlusseinrichtung ausgebildet sein kann, um das proximale Ende des Instruments und mit diesem auch den Kanal an eine Fluidquelle anzuschließen. Die Fluidquelle kann insbesondere darauf eingerichtet sein, ein gasförmiges Fluid, wie beispielsweise Luft, ein reines Gas, zum Beispiel Stickstoff, Argon, Kohlendioxid oder ein anderes gewünschtes Gas oder ein Mischgas aus einem oder mehreren Gasen zu liefern. Die Fluidquelle kann dabei weiter darauf eingerichtet sein, das gasförmige Fluid mit einem vorbestimmten Druck zu liefern, um in dem Kanal einen vorbestimmten Gasdruck und/oder eine vorbestimmte Gasströmung einzustellen. Der Instrumentenkörper kann mehrere Kanäle aufweisen, um Fluide am distalen Ende des Instruments abzugeben oder aufzunehmen. Insbesondere kann das Instrument einen oder mehrere Gaslieferkanäle und/oder einen oder mehrere Absaugkanäle aufweisen.

Der mindestens eine Kanal weist an seinem distalen Ende eine Fluidaustrittsöffnung auf. Diese Fluidaustrittsöffnung ist bei dem erfindungsgemäßen Instrument zugleich eine Lichtübertrittsöffnung, durch die Licht ein- und/oder austreten kann. Vorzugsweise ist diese Lichtübertrittsöffnung einem Wirkort des Instruments zugewandt, in welchem beispielsweise eine an dem Instrument gehaltene Elektrode angeordnet sein kann. Es können auch mehrere Elektroden vorgesehen sein. Eine oder mehrere solche Elektroden können an dem Instrument ortsfest oder beweglich angeordnet sein. Die wenigstens eine Elektrode ist außerdem an eine elektrische Quelle, zum Beispiel eine Hochfrequenzspannungsquelle anschließbar, um mittels Strom auf biologisches Gewebe einzuwirken. Insbesondere ist die elektrische Quelle darauf eingerichtet, eine Spannung und einen Strom bereitzustellen, die so groß sind, dass sie zur Funkenbildung an der Elektrode ausreichen. Die Elektrode ist beispielsweise eine Schneidelektrode, die Spannung eine Schneidspannung und der von der Elektrode zum Gewebe fließende Strom ein Schneidstrom. Der entstehende Funke emittiert Licht, das über die Lichtübertrittsöffnung in den Kanal leuchtet.

In dem Kanal ist außerdem in einem Abstand zum distalen Ende des Kanals ein optisches Element angeordnet, das ein Lichtübertrittfenster aufweist. Das Lichtübertrittsfenster und mit ihm das optische Element ist in dem Kanal unbeweglich angeordnet. Z.B. ist das optische Element mit dem Körper des Instruments unbeweglich verbunden. Außerdem ist das Lichtübertrittsfenster des optischen Elements in proximaler Richtung von der Lichtübertrittsöffnung beabstandet, die von der Gasaustrittsöffnung des Kanals gebildet ist.

Zwischen dem Lichtübertrittsfenster und der Lichtübertrittsöffnung ist eine Verschlusseinrichtung angeordnet, die ein zwischen einer Offenstellung und einer Schließstellung bewegbares Verschlussglied aufweist. In seiner Schließstellung ist das Verschlussglied dazu eingerichtet, den Kanal gegen den Durchtritt von Partikeln und insbesondere Flüssigkeitströpfchen zu sperren und somit das Lichtübertrittsfenster des optischen Elements vor Verschmutzungen abzuschirmen. Das Verschlussglied ist weiter dazu eingerichtet, den Kanal in Offenstellung sowohl für den Durchtritt von Gasen als auch für den Durchtritt von Licht freizugeben. Insbesondere ist das Verschlussglied nicht durch eine Bewegung des optischen Elements gesteuert, und zwar auch selbst dann nicht, falls dieses in den Kanal beweglich angeordnet sein sollte. Es besteht keine Wirkverbindung zwischen dem optischen Element und der Verschlusseinrichtung und insbesondere deren Verschlussglied. Das optische Element und das Verschlussglied berühren einander vorzugsweise in nicht.

Weiter vorzugsweise ist das optische Element in einem festen Abstand zu der Lichtaustrittsöffnung des Kanals angeordnet.

Das optische Element kann insbesondere eine Lichtleiteinrichtung umfassen oder durch eine Lichtleiteinrichtung gebildet sein. Beispielsweise kann das optische Element eine einzelne Lichtleitfaser oder ein Bündel von Lichtleitfasern sein, wobei die Faser oder die Fasern an ihrem jeweiligen distalen Ende jeweils ein Lichtübertrittsfenster aufweisen.

Das Lichtübertrittsfenster kann genutzt werden, um Licht zu analytischen und/oder therapeutischen Zwecken auf das zu behandelnde Gewebe zu strahlen. Das Lichtübertrittsfenster kann auch dazu genutzt werden, bei der Behandlung des Gewebes entstehendes Licht, z.B. Licht eines zwischen einer Elektrode und dem Gewebe brennenden Funkens aufzunehmen und einer Lichtanalyseeinrichtung zuzuführen. Die Lichtanalyse kann z.B. dazu dienen, das der Behandlung unterworfene Gewebe zu identifizieren. Auch andere Parameter (z.B. Funkenstabilität oder ähnliches) können erfasst werden. Zur optischen Auswertung des Lichts, bspw. Zur Gewebeanalyse, können die optische Emissionsspektroskopie (OES), optische Kohärenztomografie (OCT), Raman-Spektroskopie, Photoakustische Analysen oder andere optische Verfahren angewendet werden.

Das Verschlussglied kann in verschiedenen Ausführungsformen jeweils ein an dem Instrument ortsfest gelagertes Ende aufweisen, von dem sich der übrige Teil des Verschlussglieds weg erstreckt. Das gelagerte Ende kann mit dem Instrument fest oder beweglich, z.B. schwenkbar verbunden sein. Der übrige Teil des Verschlussglieds ist dadurch an dem Instrument beweglich gelagert und kann somit zwischen einer Offenstellung und einer Schließstellung hin und her bewegt werden. Das Verschlussglied kann starr oder elastisch, steif oder schlaff ausgebildet sein.

Es sind Ausführungsformen möglich, die mit lediglich einem einzigen Verschlussglied arbeiten. Es existieren außerdem Ausführungsformen mit mehreren Verschlussgliedern, die zum Beispiel in Offenstellung voneinander weg gespreizt und in Schließstellung miteinander in Anlage stehen. Das Verschlussglied oder die Verschlussglieder können durch biegeelastische Lamellen gebildet sein. Diese können federnd aufeinander zu vorgespannt sein, um in Schließstellung eine Kegel- oder Pyramidenform festzulegen, wobei die Berührungslinien Mantellinien des Kegels bzw. Kanten der Pyramide sind. In Offenstellung können die Lamellen voneinander weg gespreizt sein. Es ist vorteilhaft, wenn die Lamellen dazu aus biegeelastischem Material bestehen. Zusätzlich oder alternativ kann das Verschlussglied aus einem dehnbaren Material ausgebildet sein. Das Verschlussglied kann dann beispielsweise die Form eines Kegels oder einer Pyramide mit einer kleinen Öffnung an seiner bzw. ihrer Spitze aufweisen. Zum Öffnen wird das Material entsprechend gedehnt, sodass sich die Öffnung an der Kegel- oder Pyramidenspitze vergrößert. Die Basis der Pyramide oder des Kegels kann bei allen diesen Ausführungsformen dem Kanalquerschnitt entsprechen.

Vorzugsweise ist das Verschlussglied derart angeordnet, dass es in Ruhestellung den Kanal versperrt, wobei es durch den Druck und/oder die Strömung des in dem Kanal vorhandenen Fluids, insbesondere Gases, in Offenstellung überführt werden kann. Das Verschlussglied kann insoweit pneumatisch betätigbar ausgebildet sein. Der Kanal kann ein Spülgaskanal sein, wobei der von der Fluidquelle bereitgestellte Gasdruck des Spülgases oder sonstigen Gases zum Öffnen des Verschlussglieds genügt.

Vorzugsweise weist das Lichtübertrittsfenster einen Sichtkegel auf, der sich durch die Fluidaustrittsöffnung und somit durch die Lichtübertrittsöffnung erstreckt. Das Verschlussglied der Verschlusseinrichtung ist vorzugsweise so ausgebildet, dass es in Schließstellung den Sichtkegel versperrt, in Offenstellung jedoch freigibt.

Die Verschlusseinrichtung kann, wie oben beschrieben, von dem durch den Kanal geführten Fluid, insbesondere dessen Druck oder Strömungsgeschwindigkeit gesteuert sein. Das Fluid wirkt direkt auf das Verschlussglied ein, das insoweit selbstgesteuert ist. Es ist jedoch auch möglich, eine fremdgesteuerte Verschlusseinrichtung vorzusehen, die mit einer mechanischen Betätigungseinrichtung verbunden ist. Die mechanische Betätigungseinrichtung kann zum Beispiel eine elektromagnetische Betätigungseinrichtung oder eine pneumatische Betätigungseinrichtung oder eine manuelle Betätigungseinrichtung sein. Beispielsweise kann das Verschlussglied mechanisch mit einem Betätigungsknopf des Instruments versehen sein, um so jeweils kontrolliert zwischen Offenstellung und Schließstellung hin und her bewegt zu werden. Auch kann ein elektromechanischer oder ein pneumatischer Aktor zur Bewegung des Verschlussgliedes zwischen Offenstellung und Schließstellung vorgesehen sein.

Allen Ausführungsformen ist gemeinsam, dass durch die Steuerung der Verschlusseinrichtung entweder durch den Gasstrom oder durch sonstige Betätigungselemente oder -Medien eine Freigabe des Durchgangs zwischen der Lichtübertrittsöffnung und dem Lichtübertrittsfenster kontrolliert und gesteuert erfolgt. Die Freigabe, d.h. das Öffnen der Verschlusseinrichtung kann insbesondere auf Zeiten beschränkt werden, in denen tatsächlich ein Lichtübertritt erforderlich ist. Zudem kann das Instrument darauf eingerichtet sein, die Verschlusseinrichtung synchron mit der Lieferung von Fluid zum Beispiel Spülgas an den Wirkort des Instruments zu öffnen und zu schließen. Weiter kann das Instrument und/oder sein speisendes Gerät darauf eingerichtet sein, die Gaslieferung durch den Kanal vor Aktivierung der HF-Elektrode zu beginnen und erst nach Ende der Aktivierung der HF-Elektrode zu beenden. Jedenfalls aber kann sichergestellt werden, dass der Durchgang zwischen dem Lichtübertrittsfenster und der Lichtübertrittsöffnung nur freigegeben ist, wenn auch eine in distaler Richtung gerichtete Gasströmung von dem Lichtübertrittsfenster weg führt, sodass eine Verschmutzung des Lichtübertrittsfensters weitgehend ausgeschlossen ist. Bei fehlender Gasströmung schließt die Verschlusseinrichtung den Durchgang von der Lichtübertrittsöffnung zu dem Lichtübertrittsfenster und verhindert so die Verschmutzung des Lichtübertrittsfensters. Bei offener Verschlusseinrichtung wird die Verschmutzung durch die vorhandene Gasströmung verhindert, die alle verschmutzenden Partikel oder Tröpfchen von der Lichtübertrittsöffnung weg führt.

Die Verschlusseinrichtung kann ein federnd auf seine Schließstellung hin vorgespanntes Verschlussglied aufweisen, das durch einen Aktor oder die Gasströmung selbst in Offenstellung überführbar ist und überführt wird. Alternativ kann die die Absaugungseinrichtung dazu eingerichtet sein, die Verschlusseinrichtung zu betätigen. Dazu kann das Instrument einen Absaugkanal aufweisen, der in der Nähe des distalen Endes des Instruments mündet. Ein an den Absaugkanal angeschlossener Aktor ist dann dazu eigerichtet, die Verschlusseinrichtung in Abhängigkeit davon zu öffnen und zu schließen, ob in dem Absaugkanal Unterdruck herrscht oder nicht. Der Absaugkanal kann an eine Absaugeinrichtung angeschlossen sein, die darauf eingerichtet ist, synchron mit einem die Elektrode speisenden Generator aktiviert zu werden. Die Absaugeinrichtung kann alternativ dazu eingerichtet sein, vor Aktivierung des Generators aktiviert und erst nach Deaktivierung des Generators wieder deaktiviert zu werden. Ganz allgemein ist es auch möglich, sicher zu stellen, dass a) die Verschlusseinrichtung nur offen ist, während die Elektrode bestromt wird oder b) die Verschlusseinrichtung nur offen ist, während in dem Spülgaskanal ein distal gerichteter Spülgasfluss etabliert ist oder c) die Verschlusseinrichtung nur offen ist, während in dem Absagkanal eine Strömung in proximaler Richtung etabliert ist.

Weitere vorteilhafte Details der Erfindung ergeben sich aus Ansprüchen, sowie der Beschreibung und der zugehörigen Zeichnung mit ihren Figuren, die Folgendes zeigen:
Figur 1 Ein speisendes Gerät mit ein daran angeschlossenes Instrument, in Übersichtsdarstellung.
Figur 2 das distale Ende des Instruments nach Figur 1, in schematischer Querschnittsdarstellung,
Figur 3a und 3b eine schematische und ausschnittsweise Darstellung des Instruments mit Verschlusseinrichtung in geschlossenem und in offenem Zustand,
Figur 4a bis 9b weitere Ausführungsformen des Instruments und dessen Verschlusseinrichtung jeweils in Offenstellung und in geschlossener Stellung.

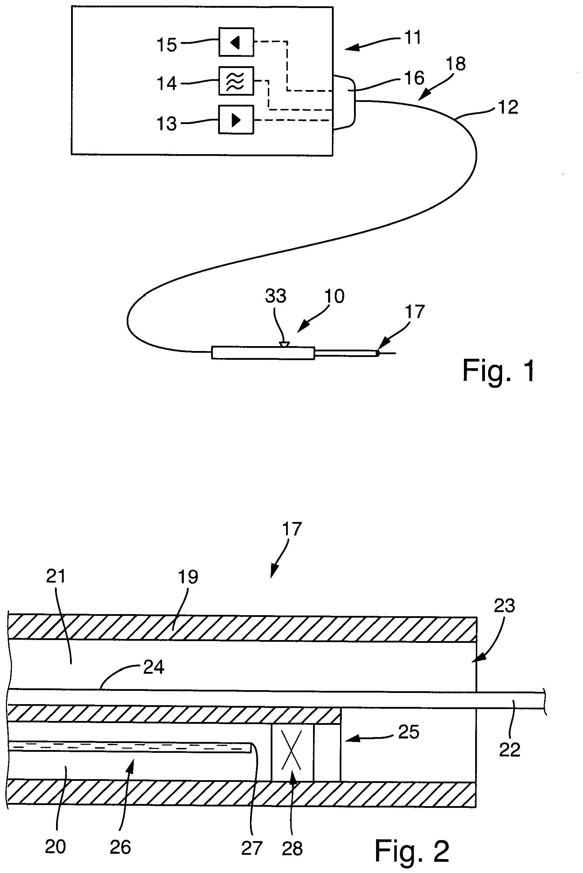

Figur 1 veranschaulicht ein erfindungsgemäßes Instrument 10, das an ein speisendes Gerät 11 angeschlossen ist. Das Gerät 11 ist über eine entsprechende Leitung 12 mit dem Instrument 10 verbunden und darauf eingerichtet, das Instrument 10 mit mindestens einem Betriebsmedium zu versorgen. Dazu kann das Gerät 11 eine oder mehrere Fluidquellen 13, einen oder mehrere elektrische Generatoren 14 und/oder eine oder mehrere Absaugeinrichtungen 15 aufweisen, die mit der Leitung 12 bzw. dem Instrument 10 über einen oder mehrere Stecker 16 verbunden sind. Figur 1 veranschaulicht das Instrument 10 als handgeführtes Instrument für den offenchirurgischen Einsatz. Die Erfindung erstreckt sich jedoch auch auf anderweitig ausgebildete Instrumente z.B. laparoskopisch einzusetzenden Instrumente oder Sonden wie sie durch den Arbeitskanal eines Endoskops, eines Trokars oder durch einen anderweitigen Zugang in den Körper eines Patienten eingeführt werden können.

Das Instrument 10 weist ein distales Ende 17 sowie ein proximales Ende 18 auf, das durch den Stecker 16 oder einen Abschnitt seiner Leitung 12 gebildet sein kann.

Das distale Ende 17 des Instruments 10 ist in Figur 2 gesondert veranschaulicht. Es beinhaltet den distalen Abschnitt eines Instrumentenkörpers 19, durch den sich mindestens ein Kanal 20 erstreckt. Der Kanal 20 ist insbesondere ein fluidführender, beispielsweiser gasführender Kanal, der sich vorzugsweise von dem distalen Ende 17 durch das Instrument 10 und seine gesamte Leitung 12 bis zu dem proximalen Ende 18 bzw. Stecker 16 erstreckt und dort an der Gasquelle 13 angeschlossen ist. Optional kann das Instrument 10 weitere Kanäle, wie beispielsweise einen Absaugkanal 21 aufweisen, der sich ebenfalls von dem distalen Ende 17 des Instruments 10 bis zu seinem proximalen Ende 18 oder Stecker 16 erstreckt und dort an die Absaugeinrichtung 15 angeschlossen ist. Optional können andere Kanäle und/oder weitere Kanäle vorgesehen sein, um Flüssigkeiten und/oder Gase an das das distale Ende 17 des Instruments 10 heran oder von diesem weg zu führen und sie dort ein- oder austreten zu lassen.

Das Instrument 10 kann Werkzeuge zur Einwirkung auf biologisches Gewebe aufweisen. Ein solches Werkzeug kann beispielsweise eine Elektrode 22 sein, die in oder an dem distalen Ende 17 des Instruments 10 gehalten ist und dort endet oder die aus einer Gasübertrittsöffnung herausschaut oder kurz vor dieser endet. Die Elektrode 22 ist über eine elektrische Leitung 24, die sich durch die Leitung 12 bis zu dem proximalen Ende 18 oder dem Stecker 16 erstreckt, mit dem Generator 14 verbunden. Es können aber auch mehrere Elektroden oder andere Werkzeuge, wie z.B. ein Wasserstrahlwerkzeug, ein Laserwerkzeug oder dergleichen vorgesehen sein.

Der Kanal 20 weist eine distale Öffnung auf, die eine Gasaustrittsöffnung und zugleich eine Lichtübertrittsöffnung 25 ist. In dem Kanal 20 ist vorzugsweise unbeweglich ein optisches Element 26, beispielsweise in Gestalt eines Lichtleiters, angeordnet, der an seinem distalen Ende ein Lichtübertrittsfenster 27 aufweist. Das Lichtübertrittsfenster 27 kann durch die Stirnfläche des optischen Elements 26, d.h. beispielsweise durch die Stirnfläche einer Lichtleitfaser, gebildet sein. Das Lichtübertrittsfenster 27 und mit ihm das optische Element 26 ist vorzugsweise axial unverschiebbar in dem Kanal 20 angeordnet. Vorzugsweise ist das Lichtübertrittsfenster 27 gegenüber der Lichtübertrittsöffnung 25 in proximaler Richtung beabstandet.

Zwischen dem Lichtübertrittsfenster 27 und der Lichtübertrittsöffnung 25 ist eine Verschlusseinrichtung 28 angeordnet. Diese Verschlusseinrichtung 28 ist dazu eingerichtet, den Durchgang von der Lichtübertrittsöffnung 25 zu dem Lichtübertrittsfenster 27 gesteuert freizugeben oder zu sperren. Vorzugsweise ist die Verschlusseinrichtung 28 von der Gasströmung in dem Kanal 20 oder auch durch andere Medien, keinesfalls aber von irgendeiner Bewegung des optischen Elements 26 gesteuert, das, wie erwähnt, vorzugsweise ortsfest angeordnet ist.

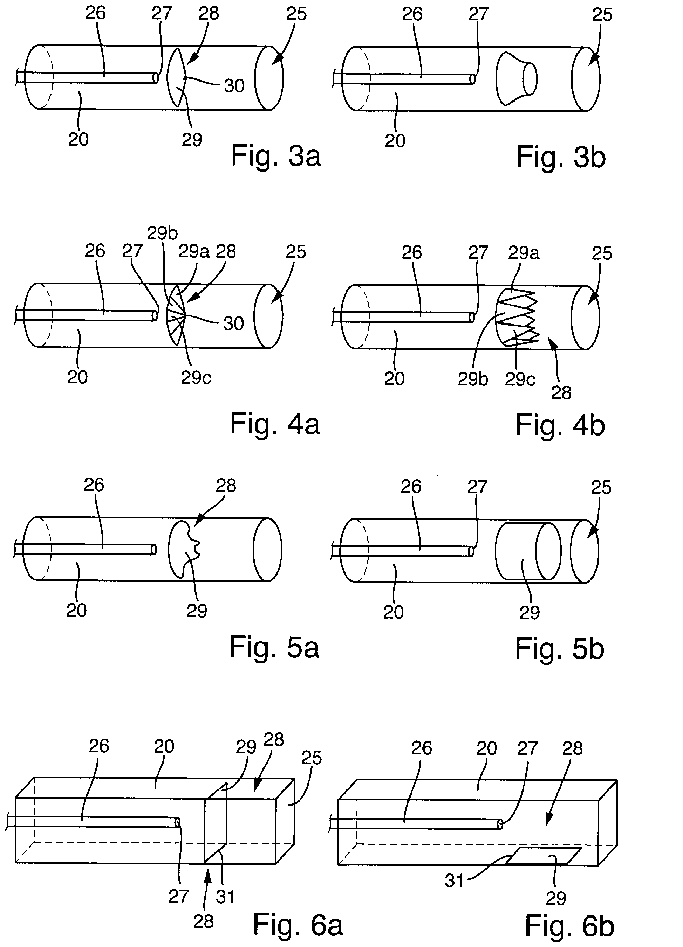

Eine erste Ausführungsform der Verschlusseinrichtung 28 geht aus den Figuren 3a und 3b hervor. Die Verschlusseinrichtung 28 wird dort durch ein ungefähr kegelförmiges Verschlussglied 29 aus zugelastischem Material gebildet, das an seiner Spitze 30 eine ausweitbare Öffnung aufweist. Die Spitze 30 ist der Lichtübertrittsöffnung 25 zugewandt. Die Öffnung an der Spitze 30 ist im Schließzustand sehr eng oder ganz geschlossen. Die Zugelastizität des Materials des Verschlussglied 29 ist so groß, dass sich die Öffnung 30 durch den in dem Kanal 20 herrschenden Druck jedoch so weit dehnen lässt, dass der gewünschte Gasstrom aus der Lichtübertrittsöffnung 25 austreten kann und somit auch der Lichtweg von der Lichtübertrittsöffnung 25 zu dem Lichtübertrittsfenster 27 freigegeben ist. Bei vorhandenem Gasstrom ist das Verschlussglied 29 in Offenstellung.

Eine abgewandelte Ausführungsform veranschaulichen die Figuren 4a und 4b für die vorige Beschreibung mit Ausnahme der nachfolgenden Besonderheiten gilt:

Die Verschlusseinrichtung 28 umfasst mehrere Verschlussglieder 29a, 29b, 29c die flexibel ausgebildet sind und, wenn sie mit ihren Kanten aneinander liegen, eine Kegel bilden. An seiner Spitze 30 treffen sich die lamellenartigen Verschlussglieder 29a, 29b, 29c usw. und verschließen somit den Durchgang von der Lichtübertrittsöffnung 25 zu dem Lichtübertrittsfenster 27. Wird der Kanal 20 hingegen mit Gas beaufschlagt, spreizen sich die Verschlussglieder 29a, 29b, 29c usw., wie es Figur 4b zeigt. Somit ist der Durchgang durch die Verschlusseinrichtung 28 sowohl für Gas als auch für Licht freigegeben, die Verschlussglieder 29a, 29b usw. sind in Offenstellung.

Das Verschlussglied 29 weist einen ortsfest an dem Instrument 10 gehaltenen Abschnitt auf. Dieser wird bei dem Verschlussglied 29 durch einen kreisförmigen Abschnitt gebildet, der den Fuß des von dem Verschlussglied 28 definierten Kegels bildet. Dieser Abschnitt ist mit der Wandung des Kanals 20 ortsfest verbunden. Die übrigen Teile des kegelförmigen Verschlussglieds 29 können elastisch oder flexibel verformt werden und sind insoweit beweglich.

In Figur 5a und 5b ist eine weiter abgewandelte Ausführungsform der Verschlusseinrichtung 28 veranschaulicht, bei der das Verschlussglied 29 aus einem faltbaren flexiblen, schlaffen Material wie beispielsweise einen Folienschlauchabschnitt gebildet ist. Dieser fällt bei fehlender Gasbeaufschlagung des Kanals 20 in sich zusammen und versperrt als unregelmäßig geformter Körper den Kanal 20. Bei Gasdurchgang entfaltet sich das Verschlussglied 29, wie in Figur 5b veranschaulicht und gibt somit den Durchgang für Licht und Gas zwischen den Lichtübertrittsfenster 27 und der Lichtübertrittsöffnung 25 frei. Ansonsten gilt die obige Beschreibung entsprechend.

Bei den beschriebenen Ausführungsformen der Figuren 3a bis 5b ist zeichnerisch ein Kanal mit rundem Querschnitt zugrunde gelegt worden. Ebenso gut kann bei diesen Ausführungsformen der Kanal 20 jedoch auch von der Kreisform abweichende Querschnitte, beispielsweise polygonale Querschnitte, wie Dreieck, Rechteck, Sechseck oder krummlinig begrenztes Mehreck, aufweisen. Rein beispielhaft wird dazu in den Figuren 6a und 6b eine Konfiguration des Kanals 20 und der Verschlusseinrichtung 28 mit rechteckigem Kanalquerschnitt veranschaulicht. Die Verschlusseinrichtung 28 umfasst hier ein als Klappe ausgebildetes Verschlussglied 29 dessen Umriss mit dem Querschnitt des Kanals 20 übereinstimmt. Das klappenartige Verschlussglied 29 ist mit einer Kante 31 mit dem Instrument bzw. der Wandung des Kanals 20 verbunden. Die Kante 31 kann dabei fest verankert und das Verschlussglied 29 flexibel ausgebildet sein. Alternativ kann die Kante 31 scharnierartig gehalten sein. In Sperrstellungnach Figur 6a versperrt das Verschlussglied 29 den Kanal 20 und somit den Durchgang zwischen dem Lichtübertrittsfenster 27 und der Lichtaustrittsöffnung 25. Das Verschlussglied 29 kann auf diese Position hin federnd vorgespannt sein. Diese federnde Vorspannung kann durch die Eigenelastizität des Verschlussglieds 29 oder durch ein gesondertes Federelement erbracht sein. Dem Verschlussglied 29 kann ein Anschlag zugeordnet sein, an dem es in Sperrstellung anliegt (nicht dargestellt). Dies ist insbesondere bei Ausführungsformen vorteilhaft, bei denen das Verschlussglied 29 an der Kante 31 durch ein Scharnier gehalten ist. In Figur 6b ist die Offenposition der Verschlusseinrichtung 28 veranschaulicht, bei der das Verschlussglied 29 durch das den Kanal 20 in Distalrichtung durchströmende Gas aus seiner Ruhelage heraus in die Offenstellung gedrängt ist.

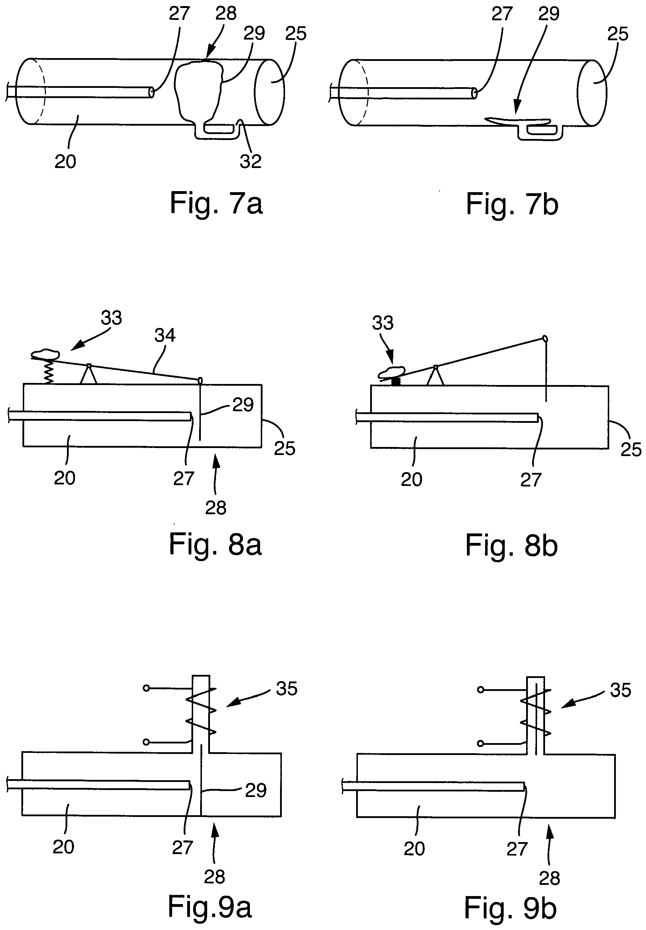

Figur 7a und 7b veranschaulichen eine weitere Ausführungsform einer fluidbetätigten Verschlusseinrichtung 28. Bei dieser gehört zu der Verschlusseinrichtung 28 ein ballonartiges hohles Verschlussglied 29, das wiederum zwischen dem Lichtübertrittsfenster 27 und der Lichtübertrittsöffnung 25 angeordnet ist. Das ballonartige Verschlussglied 29 steht in Fluidverbindung mit einer im Gasweg angeordneten Venturiöffnung 32, über die der statische Druck der Gasströmung wirksam wird. Nimmt der statische Druck infolge der Geschwindigkeit der Gasströmung in dem Kanal 20 ab, führt dies zum Kollabieren des ballonartigen Verschlussglieds 29, sodass, wie in Figur 7b dargestellt, das Verschlussglied 29 den Gasweg freigibt.

Bei einer favorisierten Ausführungsform ist das ballonartige Verschlussglied 29 oder ein Aktor zur Betätigung des Verschlussglieds 29 an den Absaugkanal 21 angeschlossen. Dadurch kann der Unterdruck der Absaugung zur Betätigung der Verschlusseinrichtung 28 genutzt werden. Dazu kann eine Querbohrung auf Höhe von Element 28 zu Element 21 (Figur 2) vorgesehen werden. Es wird damit ein robustes System geschaffen. Insbesondere können größere Druckdifferenzen und somit große Stellkräfte erzeugt werden.

Bei einer solchen Ausführungsform sind vorzugsweise die Absaugeinrichtung 15 und die Spülgasquelle 13 mit dem HF-Generator 14 synchronisiert. Dies reduziert die Schalldruckbelastung, die ansonsten bei Dauerbetrieb zu hoch wäre.

Alle vorstehend beschriebenen Ausführungsformen haben gemeinsam, dass das Verschlussglied 29 der Verschlusseinrichtung 28 pneumatisch durch die Wirkung des Gases bzw. seiner Strömung in dem Kanal 20 gesteuert ist. Es ist jedoch auch möglich, die Verschlusseinrichtung 28 fremd zu steuern, wie es die Figuren 8a und 8b in einem einfachen ersten Beispiel zeigen. Das Instrument 10 weist dort eine lediglich schematisch veranschaulichte Betätigungseinrichtung 33 auf, die beispielsweise durch einen Bedienknopf an dem Instrument 10 gebildet sein kann. Die Betätigungseinrichtung 33 kann dabei dazu vorgesehen sein, das Instrument 10 und somit den angeschlossenen Generator 11 zu aktivieren oder zu deaktivieren. Die Betätigungseinrichtung 33 kann zusätzlich oder alternativ auch zur Auslösung anderer Aktivierungs- oder Schaltvorgänge ausgebildet sein, z.B. zum Auslösen einer Spektraluntersuchung des von dem Funken der Elektrode ausgehenden Lichts. Zugleich kann die Betätigungseinrichtung über ein geeignetes, hier lediglich schematisch durch einen zweiarmigen Hebel 34 veranschaulichtes Getriebe das Verschlussglied 29 bewegen, sodass es in Schließstellung nach Figur 8a den Durchgang zwischen dem Lichtübertrittsfenster 27 und der Lichtübertrittsöffnung 25 sperrt und in Betätigungsstellung gemäß Figur 8b freigibt.

Die Betätigung der Verschlusseinrichtung 28 kann, wie es am Beispiel der Figuren 9a und 9b ersichtlich wird, auch durch anderweitige Aktoren beispielsweise durch einen magnetischen Aktor 35 erfolgen, mit dem das Verschlussglied 29 in Schließstellung nach Figur 9a in dem Kanal 20 und in Offenstellung, wie es Figur 9b zeigt, außerhalb des Kanals 20 positioniert sein kann.

Das erfindungsgemäße Instrument 10 nach Figur 1 arbeitet wie folgt:
Zur Anwendung an einem Patienten wird das Instrument 10 an den Operationsort herangeführt und durch Betätigung eines geeigneten Schalters zum Beispiel der Betätigungseinrichtung 33 aktiviert. Zur Aktivierung können alternativ auch andere Schaltmittel beispielsweise Fußschalter oder sonstige Bedieneinrichtungen dienen. Das Gerät 11 erhält dadurch einen entsprechenden Schaltimpuls und aktiviert die jeweils angesprochenen bzw. vorhandenen Einrichtungen, wie die Gasquelle 13, den elektrischen Generator 14 und/oder die Absaugeinrichtung 15. Handelt es sich bei dem Instrument 10 um ein elektrochirurgisches Instrument, wird typischerweise die Gasquelle 13 aktiviert bevor der Generator 14 aktiviert wird. Solange die Gasquelle 13 nicht aktiviert ist, d.h. den Kanal 20 nicht mit Gas beaufschlagt ist, ist die Verschlusseinrichtung 28 geschlossen. Sobald entsprechend ausreichender Gasdruck in dem Kanal 20 und/oder eine Gasströmung in dem Kanal 20 etabliert ist, öffnet die Verschlusseinrichtung 28, jedenfalls wenn eine der Ausführungsformen nach den Figuren 3a bis 7b Anwendung findet.

Nach Aktivierung des Generators 14 wird von der Elektrode 22 ausgehendes Licht von dem optischen Element 26 erfasst. Die Elektrode 22 liegt vorzugsweise im Sichtbereich des optischen Elements 26, d.h. im Sichtkegel, der von dem Lichtübertrittsfenster 27 ausgeht und sich durch die Lichtübertrittsöffnung 25 erstreckt. So kann von der Elektrode ausgehendes Funkenlicht erfasst und von dem optischen Element 26 an eine Analyseeinrichtung beispielsweise einen Spektralanalysator weitergeleitet werden, der Teil des Instruments 11 sein oder separat ausgebildet und aufgestellt sein kann.

Der durch den Kanal 20 vordringende und aus der Lichtübertrittsöffnung 25 ausströmende Gasstrom verhindert wirksam einen Niederschlag von Partikeln, Tröpfchen oder sonstigen Verschmutzungen auf dem Lichtübertrittsfenster 27. Sobald jedoch der Gasstrom zum Erliegen kommt, beispielsweise, weil er unterbrochen wird, schließt die Verschlusseinrichtung 28. Selbst wenn nun weiterhin Verschmutzungen durch Aufwirbelungen oder dergleichen entstehen, beispielsweise durch die Aktivität der Absaugeinrichtung 25, die über den Absaugkanal 21 Flüssigkeiten oder sonstige Materie aus dem Operationsgebiet absaugt, bleibt das Lichtübertrittsfenster 27 abgeschottet und von Verschmutzungen verschont. Dies gilt auch, wenn ohne Gaszutritt durch den Kanal 20 mit der Elektrode 22 weitergearbeitet wird und auf diese Weise Partikel freigesetzt werden.

Die in den Figuren 8a bis 9b veranschaulichten Ausführungsformen des Instruments 10 arbeiten auf ähnliche Weise. Bei der Ausführungsform nach Figur 8a und 8b wird die Verschlusseinrichtung 28 immer dann freigegeben, wenn die Betätigungseinrichtung 33 aktiviert wird. Die Aktivierung der Betätigungseinrichtung 33 kann unabhängig von der Aktivierung des Geräts 11 und des Generators 14 stattfinden. Beispielsweise kann der Kanal 20 mit unter geringem Druck stehendem Gas beaufschlagt sein. Bei geschlossener Verschlusseinrichtung 28 kann diese zugleich den Gasstrom und den Lichtdurchtritt blockieren. Ebenso blockiert die Verschlusseinrichtung 28 den Durchtritt von Verschmutzungen. Sobald die Verschlusseinrichtung 28 mittels der Betätigungseinrichtung 33 geöffnet wird, wird der Lichtweg freigegeben. Zugleich wird der Gasstrom freigegeben, der ein Eindringen von Verschmutzungen verhindert. Soll ain Gasstrom auch dann möglich sein, wenn die Verschlusseinrichtung 28 geschlossen ist, kann parallel zu der Verschlusseinrichtung 28 ein Bypass vorgesehen sein, der für Licht undurchlässig ist.

Bei der Ausführungsform nach Figur 9a und 9b wird die Verschlusseinrichtung 28 durch den magnetischen Aktor 35 oder einen anderen geeigneten elektrischen Aktor betätigt. Die Bestromung des Aktors 35 kann synchron mit der Bestromung der Elektrode 22 oder unabhängig von dieser erfolgen. Letzteres beispielsweise, wenn lediglich gelegentlich Licht von der Elektrode 22 aufgenommen werden soll. Das Gerät 11 ist dann in Verbindung mit dem Instrument 10 si ausgebildet, das zur Aufnahme von Licht von dem Behandlungsort, beispielsweise zur Spektralanalyse des Lichts, das Spektrometer und der Aktor 35 gleichzeitig aktiviert werden. Damit ist das Lichtübertrittsfenster 27 dauernd vor Verschmutzung geschützt. Im aktivierten Zustand verhindert der durch den Kanal 20 strömende Gasfluss eine Verschmutzung. Bei Deaktivierung schließt die Verschlusseinrichtung 28 und schließt das Lichtübertrittsfenster 27. Dieses Prinzip gilt für alle Ausführungsformen.

Die Erfindung ist nicht auf elektrochirurgische Instrumente beschränkt. Sie kann auch für laserchirurgische Instrumente oder kryochirurgische Instrumente angewendet werden. Im laserchirurgischen Fall kann das optische Element 26 dazu ausgebildet sein, einen Laserstahl aus dem Lichtübertrittsfenster 27 durch die Lichtübertrittsöffnung 25 zum Gewebe zu senden. Der Gasstrom in dem Kanal 20 hält im aktivierten Falle auch hier das Lichtübertrittsfenster 27 von Verschmutzungen frei. Bei Deaktivierung schließt die Verschlusseinrichtung 28 beispielsweise nach den Prinzipien der Figuren 8a bis 9b.

Das Instrument 10 kann auch ein wasserstrahlchirurgisches oder anderweitiges Instrument sein. Zusätzlich oder alternativ zu der Elektrode 22 kann dann ein Wasserinjektionskanal vorgesehen sein, um entsprechendes Gewebe am Operationsort vorzugsweise im Blickfeld des optischen Elements 26 zu behandeln. Die vorige Beschreibung gilt im Übrigen entsprechend.

Ein erfindungsgemäßes Instrument zur medizinischen oder chirurgischen Behandlung eines menschlichen oder tierischen Patienten weist mindestens ein zur Einwirkung auf einen Patienten geeignetes Werkzeug, beispielsweise eine Elektrode 22 auf, das bzw. die im Blickfeld eines optischen Elements 26 liegt. Das optische Element 26 ist in einem Kanal 20 angeordnet, in dem eine in distaler Richtung gerichtete Fluidströmung aufrechterhalten oder hervorrufbar ist. Das Lichtübertrittsfenster 27 des optischen Elements 26 ist gegenüber der distalen Öffnung 25 des Kanals 22 in proximaler Richtung zurück versetzt. Zwischen dem Lichtübertrittsfenster 27 und der Öffnung 25 des Kanals 20 ist eine Verschlusseinrichtung 28 angeordnet, die den Stoffdurchtritt, insbesondere den Durchtritt von Flüssigkeitstropfen und Partikeln, von der Öffnung 25 zu dem Lichtübertrittsfenster 27 blockiert, solange sie in Schließstellung steht. Ist die Verschlusseinrichtung 28 offen, gibt sie den Fluidweg und den Lichtweg zwischen dem Lichtübertrittsfenster 27 zu der Öffnung 25 frei. Vorzugsweise wird die Verschlusseinrichtung 28 pneumatisch durch das in dem Kanal 20 strömende flüssige oder gasförmige Fluid gesteuert. Mit dieser Maßnahme lässt sich eine Verschmutzung des Lichtübertrittsfensters 27 während des Betriebs des Instruments 10 sicher ausschließen oder wenigstens auf ein geringes Maß reduzieren.

### Bezugszeichen:

- 10: Instrument
- 11: Gerät
- 12: Leitung
- 13: Gasquelle
- 14: elektrischer Generator
- 15: Absaugeinrichtung
- 16: Stecker
- 17: distales Ende des Instruments 10
- 18: proximales Ende des Instruments 10
- 19: Instrumentenkörper
- 20: Kanal
- 21: Absaugkanal
- 22: Elektrode
- 23: Gasübertrittsöffnung
- 24: elektrische Leitung
- 25: distale Öffnung des Kanals 20, Lichtübertrittsöffnung
- 26: optisches Element / Lichtleitfaser
- 27: Lichtübertrittsfenster
- 28: Verschlusseinrichtung
- 29: Verschlussglied
- 30: Spitze des Verschlussglied 29
- 31: Kante des Verschlussglied 29 in Figur 6a, 6b
- 32: Venturiöffnung
- 33: Betätigungseinrichtung
- 34: Hebel
- 35: Aktor

## Patentansprüche

1. Instrument (10) zur medizinischen oder chirurgischen Behandlung eines menschlichen oder tierischen Patienten,
mit einem Instrumentenkörper (19), in oder an dem mindestens ein Kanal (20) ausgebildet ist, der ein distales Ende (17) und ein proximales Ende (18) aufweist,
wobei der Kanal (20) an seinem proximalen Ende (18) an eine Fluidquelle (13) anschließbar ist und an seinem distalen Ende eine Öffnung (25) aufweist,
mit einem optischen Element (26), das wenigstens ein in dem Kanal (20) ortsfest angeordnetes Lichtübertrittsfenster (27) aufweist,
mit einer Verschlusseinrichtung (28), die in dem Kanal (20) zwischen dem Lichtübertrittsfenster (27) und dem distalen Ende (17) des Fluidkanals (20) angeordnet ist und wenigstens ein Verschlussglied (29) aufweist, das zwischen einer Offenstellung und einer Schließstellung bewegbar ist,
**dadurch gekennzeichnet, dass** das Verschlussglied (29) derart angeordnet ist, dass es durch in dem Fluidkanal (20) strömendes Fluid betätigbar ist.

2. Instrument nach Anspruch 1, **dadurch gekennzeichnet, dass** das Verschlussglied (29) ein an dem Instrument (10) ortsfest gelagertes Ende aufweist.

3. Instrument nach einem der vorstehenden Ansprüche, **dadurch gekennzeichnet, dass** die Verschlusseinrichtung (28) mehrere Verschlussglieder (29a, 29b) aufweist, die jeweils an einem Ende an dem Instrument (10) schwenkbar gelagert sind.

4. Instrument nach vorstehenden Ansprüche, **dadurch gekennzeichnet, dass** die Verschlusseinrichtung (28) mehrere biegeelastische Lamellen aufweist.

5. Instrument nach einem der vorstehenden Ansprüche, **dadurch gekennzeichnet, dass** das Verschlussglied (29) ein aus einem dehnbaren Material ausgebildetes Element ist.

6. Instrument nach einem der vorstehenden Ansprüche, **dadurch gekennzeichnet, dass** der Kanal (20) an eine Gasquelle (13) als Fluidquelle anschließbar ist.

7. Instrument nach einem der vorstehenden Ansprüche, **dadurch gekennzeichnet, dass** das Instrument (10) wenigstens einen weiteren Kanal (20) zur Zu- oder Abführung von Fluiden aufweist.

8. Instrument nach einem der vorstehenden Ansprüche, **dadurch gekennzeichnet, dass** von dem Lichtübertrittsfenster (27) ein Sichtkegel festgelegt ist, der sich durch die Öffnung (25) erstreckt.

9. Instrument nach einem der vorstehenden Ansprüche, **dadurch gekennzeichnet, dass** das Instrument (10) eine Elektrode (11) aufweist.

10. Instrument nach Anspruch 9, **dadurch gekennzeichnet, dass** die Elektrode (22) in oder vor der Öffnung (25) angeordnet ist.

11. Instrument nach den Ansprüchen 8 und 9, **dadurch gekennzeichnet, dass** die Elektrode (22) wenigstens teilweise in dem Sichtkegel angeordnet ist.

12. Instrument nach einem der vorstehenden Ansprüche, **dadurch gekennzeichnet, dass** die Verschlusseinrichtung (28) mit einer mechanischen Betätigungseinrichtung (33) verbunden ist.

13. Instrument nach einem der vorstehenden Ansprüche, **dadurch gekennzeichnet, dass** die Verschlusseinrichtung (28) mit einer elektromagnetischen Betätigungseinrichtung (35) verbunden ist.

14. Instrument nach einem der vorstehenden Ansprüche, **dadurch gekennzeichnet, dass** die Verschlusseinrichtung (28) mit einer pneumatischen Venturi-Betätigungseinrichtung (32) verbunden ist.

## Claims

1. An instrument (10) for medical or surgical treatment of a human or animal patient,
having an instrument body (19) in or on which at least one channel (20) is formed that comprises a distal end (17) and a proximal end (18),
wherein the channel (20) is connectable to a fluid source (13) at its proximal end (18) and comprises an opening (25) at its distal end,
having an optical element (26) that comprises at least one light passage window (27) that is immovably arranged in the channel (20),
having a closure device (28) that is arranged in the channel (20) between the light passage window (27) and the distal end (17) of the fluid channel (20) and that comprises at least one closure member (29) which is movable between an open position and a closed position,
**characterized in that** the closure member (29) is arranged such that it can be actuated by fluid flowing in the fluid channel (20)

2. The instrument according to claim 1, **characterized in that** the closure member (29) comprises an end fixedly mounted on the instrument (10).

3. The instrument according to anyone of the preceding claims, **characterized in that** the closure device (28) comprises multiple closure members (29a, 29b) that are respectively pivotably mounted at one end on the instrument (10).

4. The instrument according to anyone of the preceding claims, **characterized in that** the closure device (28) comprises multiple bending elastic lamellae.

5. The instrument according to anyone of the preceding claims, **characterized in that** the closure member (29) is an element made of a stretchable material.

6. The instrument according to anyone of the preceding claims, **characterized in that** the channel (20) can be connected to a gas source (13) as a fluid source.

7. The instrument according to anyone of the preceding claims, **characterized in that** the instrument (10) comprises at least one additional channel (20) for supply or discharge of fluids.

8. The instrument according to anyone of the preceding claims, **characterized in that** a sight cone is defined by the light passage window (27) that extends through the opening (25).

9. The instrument according to anyone of the preceding claims, **characterized in that** the instrument (10) comprises an electrode (11).

10. The instrument according to claim 9, **characterized in that** the electrode (22) is arranged in or in front of the opening (25).

11. The instrument according to claims 8 and 9, **characterized in that** the electrode (22) is arranged at least partly within the sight cone.

12. The instrument according to anyone of the preceding claims, **characterized in that** the closure device (28) is connected to a mechanical actuating device (33).

13. The instrument according to anyone of the preceding claims, **characterized in that** the closure device (28) is connected to an electromagnetic actuating device (35).

14. The instrument according to anyone of the preceding claims, **characterized in that** the closure device (28) is connected to a pneumatic Venturi actuating device (32).

## Revendications

1. Instrument (10) pour le traitement médical ou chirurgical d'un patient humain ou animal,
avec un corps d'instrument (19), dans ou sur lequel est réalisé au moins un canal (20), qui présente une extrémité distale (17) et une extrémité proximale (18),
dans lequel le canal (20) peut être raccordé à une source de fluide (13) à son extrémité proximale (18) et présente une ouverture (25) à son extrémité distale,
avec un élément optique (26), qui présente au moins une fenêtre de passage de lumière (27) disposée de manière fixe dans le canal (20),
avec un dispositif de fermeture (28), qui est disposé dans le canal (20) entre la fenêtre de passage de lumière (27) et l'extrémité distale (17) du canal de fluide (20) et présente au moins un organe de fermeture (29), qui est mobile entre une position ouverte et une position fermée, **caractérisé en ce que** l'organe de fermeture (29) est disposé de telle sorte qu'il peut être actionné par le fluide circulant dans le canal de fluide (20).

2. Instrument selon la revendication 1, **caractérisé en ce que** l'organe de fermeture (29) présente une extrémité montée fixe sur l'instrument (10).

3. Instrument selon l'une quelconque des revendications précédentes, **caractérisé en ce que** le dispositif de fermeture (28) présente plusieurs organes de fermeture (29a, 29b) qui sont respectivement montés de manière pivotante sur l'instrument (10) à une extrémité.

4. Instrument selon les revendications précédentes, **caractérisé en ce que** le dispositif de fermeture (28) présente plusieurs lamelles élastiques en flexion.

5. Instrument selon l'une quelconque des revendications précédentes, **caractérisé en ce que** l'organe de fermeture (29) est un élément réalisé à partir d'un matériau extensible.

6. Instrument selon l'une quelconque des revendications précédentes, **caractérisé en ce que** le canal (20) peut être raccordé à une source de gaz (13) en tant que source de fluide.

7. Instrument selon l'une quelconque des revendications précédentes, **caractérisé en ce que** l'instrument (10) présente au moins un autre canal (20) pour l'amenée ou l'évacuation de fluides.

8. Instrument selon l'une quelconque des revendications précédentes, **caractérisé en ce qu'**un cône de vision, qui s'étend à travers l'ouverture (25), est défini par la fenêtre de passage de lumière (27).

9. Instrument selon l'une quelconque des revendications précédentes, **caractérisé en ce que** l'instrument (10) présente une électrode (11).

10. Instrument selon la revendication 9, **caractérisé en ce que** l'électrode (22) est disposée dans ou devant l'ouverture (25).

11. Instrument selon les revendications 8 et 9, **caractérisé en ce que** l'électrode (22) est disposée au moins en partie dans le cône de vision.

12. Instrument selon l'une quelconque des revendications précédentes, **caractérisé en ce que** le dispositif de fermeture (28) est relié à un dispositif d'actionnement mécanique (33).

13. Instrument selon l'une quelconque des revendications précédentes, **caractérisé en ce que** le dispositif de fermeture (28) est relié à un dispositif d'actionnement électromagnétique (35).

14. Instrument selon l'une quelconque des revendications précédentes, **caractérisé en ce que** le dispositif de fermeture (28) est relié à un dispositif d'actionnement venturi pneumatique (32).
